# EUROPEAN PATENT SPECIFICATION

(11) **EP 0 897 984 B1**
(45) Date of publication and mention of the grant of the patent: **23.04.2008**
(21) Application number: 98115231.7
(22) Date of filing: 13.08.1998
(51) Int. Cl.: C12N 15/53, C12N 9/04, C12P 19/02, C12N 1/21

(54) **D-Sorbitol dehydrogenase gene**
D-Sorbitoldehydrogenase Gen
Gène de deshydrogenase de sorbitol

(30) Priority: 21.08.1997 EP 97114432
(43) Date of publication of application: 24.02.1999
(73) Proprietor: DSM IP Assets B.V., 6411 TE Heerlen (NL)
(72) Inventor: Hoshino, Tatsuo, Kamakura-shi, Kanagawa-ken (JP); Ojima, Setsuko, Fujisawa-shi, Kanagawa-ken (JP); Shinjoh, Masako, Kamakura-shi, Kanagawa-ken (JP); Tomiyama, Noribumi, Fujisawa-shi, Kanagawa-ken (JP); Miyazaki, Taro, Fujisawa-shi, Kanagawa-ken (JP)
(74) Representative: Seibel-Thomsen, Nadja

(56) References cited:
- NG KENNETH ET AL: "Sorbitol dehydrogenase from Bacillus subtilis: Purification, characterization, and gene cloning" JOURNAL OF BIOLOGICAL CHEMISTRY, AMERICAN SOCIETY OF BIOLOGICAL CHEMISTS, BALTIMORE, MD, US, vol. 267, no. 35, 1992, pages 24989-24994, XP002151857 ISSN: 0021-9258
- CHOI E-S ET AL: "PURIFICATION OF A MEMBRANE-BOUND SORBITOL DEHYDROGENASE FROM GLUCONOBACTER SUBOXYDANS" FEMS MICROBIOLOGY LETTERS, AMSTERDAM, NL, vol. 125, no. 1, 1995, pages 45-49, XP000852950 ISSN: 0378-1097
- KONDO K ET AL: "CHARACTERIZATION OF THE GENES ENCODING THE THREE-COMPONENT MEMBRANE-BOUND ALCOHOL DEHYDROGENASE FROM GLUCONOBACTER SUBOXYDANS AND THEIR EXPRESSION IN ACETOBACTER PASTEURIANUS" APPLIED AND ENVIRONMENTAL MICROBIOLOGY, WASHINGTON,DC, US, vol. 63, no. 3, March 1994 (1994-03), pages 1131-1138, XP002914612 ISSN: 0099-2240
- SHINJOH, M ET AL: "Main Polyol Dehydrogenase of Gluconobacter suboxydans IFO 3255, Membrane-bound D-Sorbitol Dehydrogenase, That Needs Product of Upstream Gene, sldB, for Activity" BIOSCI. BIOTECHNO. BIOCHEM., vol. 66, no. 1, 2002, pages 2314-2322,
- SUGISAWA T AND HOSHINO T: "Purification and Properties of Membrane-bound D-Sorbitol Dehydrogenase from Gluconobacter suboxydans IFO 3255" BIOSCI. BIOTECHNOL. BIOCHEM., vol. 66, no. 11, 2002, pages 57-64,

## Description

The present invention relates to a novel DNA coding for sorbitol dehydrogenase of a microorganism belonging to acetic acid bacteria including the genus *Gluconobacter* and the genus *Acetobacter*, an expression vector containing the said DNA, and recombinant organisms containing the said expression vector. Furthermore, the present invention relates to a process for producing recombinant D-sorbitol dehydrogenase and a process for producing L-sorbose by utilizing the said recombinant enzymes or recombinant organisms containing the said expression vector.

L-Sorbose is an important intermediate in the actual industrial process of vitamin C production, which is mainly practiced by Reichstein method (Helvetica chimica Acta, 17: 311, 1934). In the process, the only microbial conversion is the L-sorbose production from D-sorbitol by *Gluconobacter* or *Acetobacter* strains. The conversion is considered to be carried out by NAD/NADP independent D-sorbitol dehydrogenase (SLDH). L-Sorbose is also a well known substrate in the art for microbiologically producing 2-keto-L-gulonic acid, which is a useful intermediate in the production of vitamin C.

It is known that there are NAD/NADP-independent D-sorbitol dehydrogenase which catalyzes the oxidation of D-sorbitol to L-sorbose. The said D-sorbitol dehydrogenase was isolated and characterized from *Gluconobacter suboxydans* var α IFO 3254 (E. Shinagawa et al., Agric Biol. Chem., 46: 135-141, 1982), and found to consist of three subunits with the molecular weight of 63 kDa, 51 kDa, and 17 kDa; the largest subunit is dehydrogenase containing FAD as a cofactor, the second one is cytochrome c AB/vs / 26.6.98 and the smallest one is a protein with unknown function; and shows its optimal pH at 4.5. Such SLDH was also purified and characterized from *G*. *suboxydans* ATCC 621 (KCTC 2111) (E-S Choi et al., FEMS Microbiol. Lett. 125:45-50, 1995) and found to consist of three subunits with the molecular weight of 75 kDa, 50 kDa, and 14 kDa; the large subunit is dehydrogenase containing pyrroloquinoline quinone (PQQ) as a cofactor, the second one is cytochrome *c* and the small one is a protein with unknown function. The inventors also purified and characterized the NAD/NADP-independent SLDH from *G*. *suboxydans* IFO 3255 (T. Hoshino et al., EP 728840); the SLDH consists of one kind of subunit with the molecular weight of 79.0 +/- 0.5 kDa and shows its optimal pH at 6 to 7 and shows dehydrogenase activity on mannitol and glycerol as well as on D-sorbitol.

Although several SLDHs have been purified, their genes have not been cloned yet. It is useful to clone the SLDH gene for efficient production of the SLDH enzyme and for constructing recombinant organism having enhanced SLDH activity to improve the production yield of L-sorbose. It is also useful to introduce the said SLDH gene into desired organisms, for example, *Gluconobacter* converting L-sorbose to 2-keto-L-gulonic acid for constructing recombinant microorganisms which directly produce 2-keto-L-gulonic acid from D-sorbitol.

The present invention provides a nucleotide sequence (gene) coding for D-sorbitol dehydrogenase (SLDH) originating from a microorganism belonging to acetic acid bacteria including the genus *Gluconobacter* and the genus *Acetobacter*; a DNA molecule comprising said nucleotide sequence as well as a combination of the said DNA with a DNA comprising a nucleotide sequence of a protein functional in activating the said SLDH in vivo, expression vectors carrying the DNA comprising SLDH nucleotide sequence or the said combination of the DNAs; recombinant organisms carrying the expression vectors; a process for producing the recombinant SLDH; and a process for producing L-sorbose by utilizing the recombinant SLDH and a protein which is functional inactivating SLDH in vivo, or the recombinant organisms.

Furthermore the present invention is directed to DNA sequences encoding the polypeptides with SLDH activity and polypeptides functional in activating the said SLDH in vivo as disclosed e.g. in the sequence list as SEQ ID NO:2 and NO:3 as well as their complementary strands, or those which include these sequences, DNA sequences which hybridize under stringent conditions with such sequences.

A man skilled in the art is familiar with stringent hybridization and stringent washing conditions, which are described, e.g. in Sambrook et al., Molecular Cloning, Cold Spring Harbor Laboratory Press, New York, USA, second edition 1989.

In the following a brief description of the drawings is given.

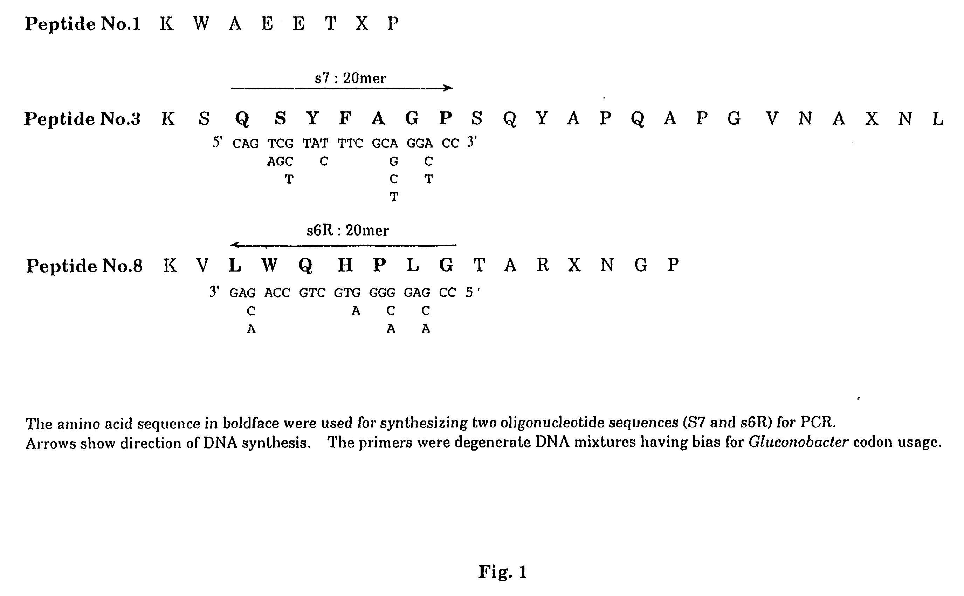

Figure 1 illustrates the partial amino acid sequences of SLDH polypeptide and oligonucleotides. The amino acid sequences in boldface in the figure were used for synthesizing two oligonucleotide sequences (S7 and S6R) for PCR. Arrow shows direction of DNA synthesis. All primers were degenerate DNA mixtures having bias for *Gluconobacter* codon usage.

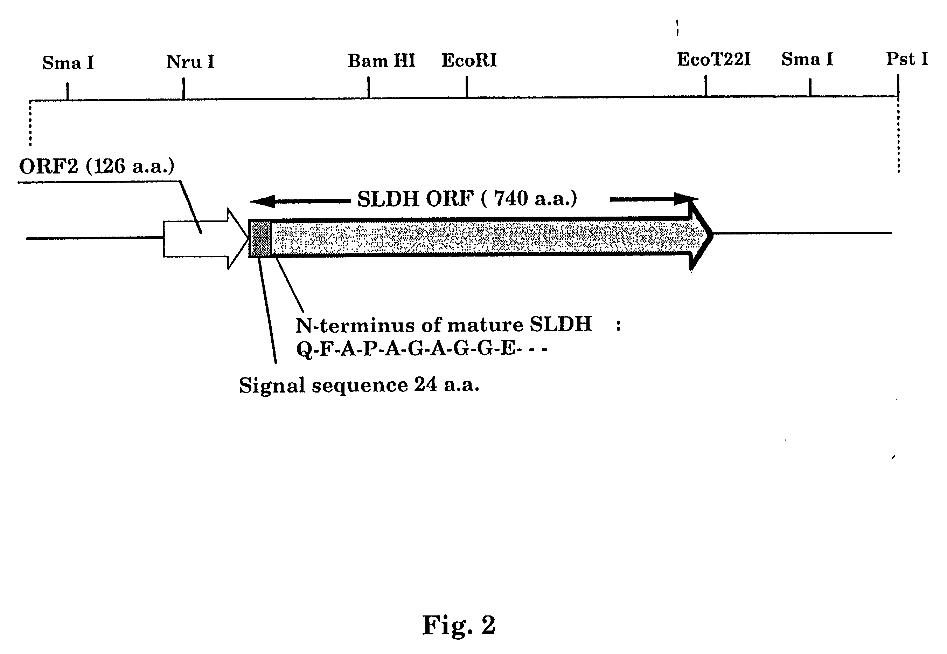

Figure 2 illustrates a restriction map of the SLDH gene cloned in the present invention and the genetic structure of the DNA region encoding SLDH and ORF2.

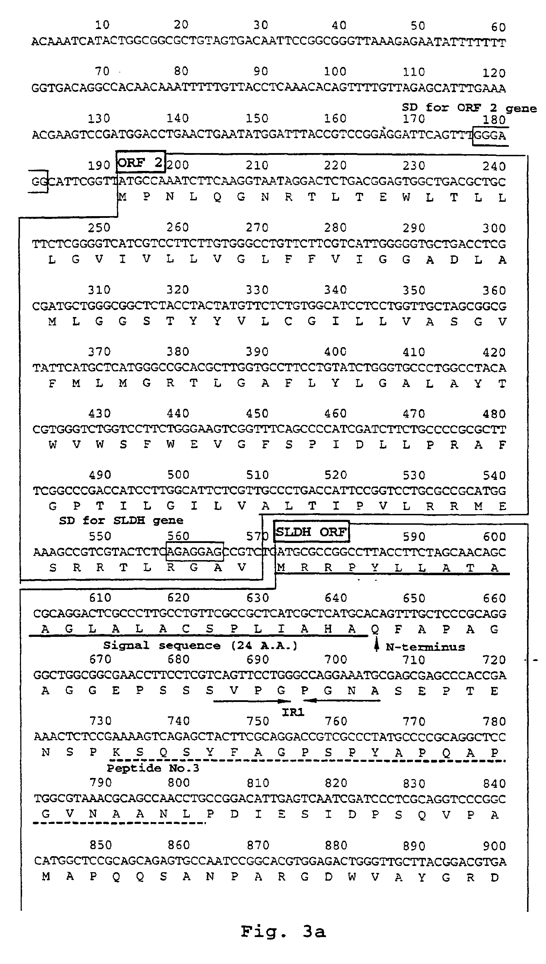

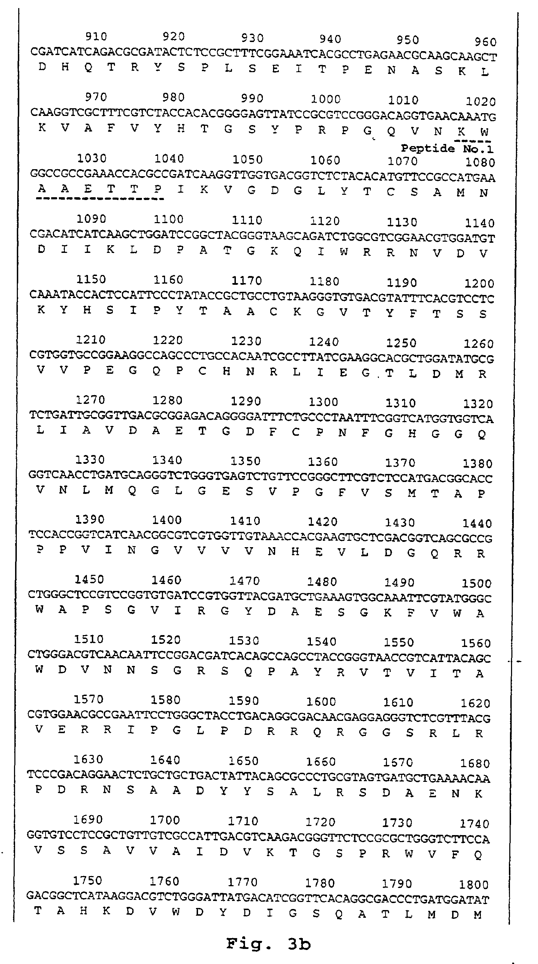

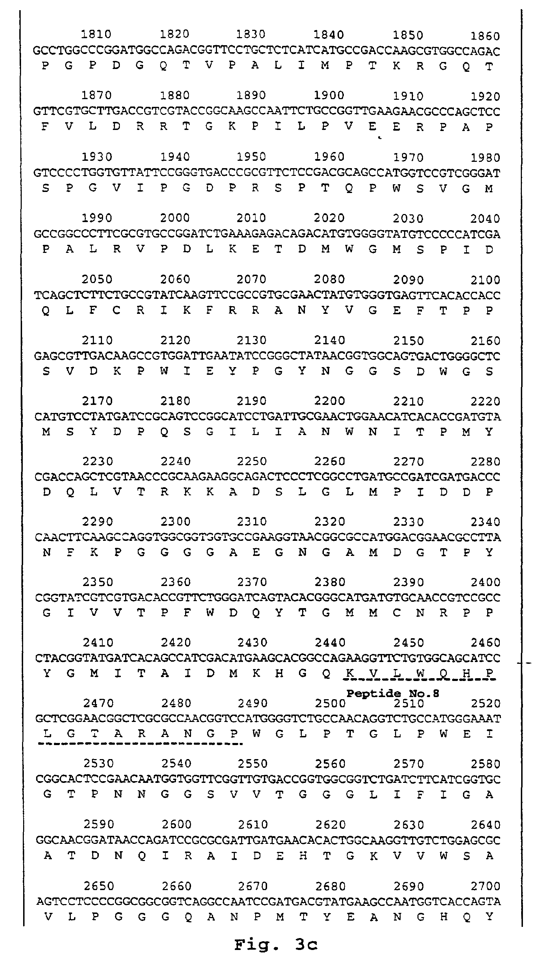

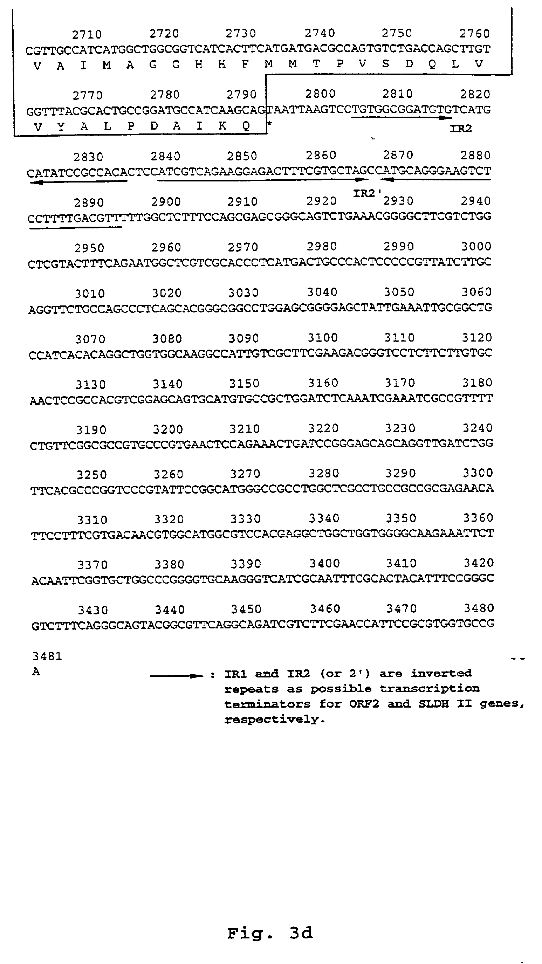

Figure 3 illustrates the nucleotide sequence encoding SLDH and ORF2 with upstream and downstream sequences and illustrates deduced amino acid sequences of SLDH and ORF2. Figure 3 also illustrates putative ribosome-binding sites (SD sequences) of SLDH and ORF2 genes and the possible transcription terminator sequences.

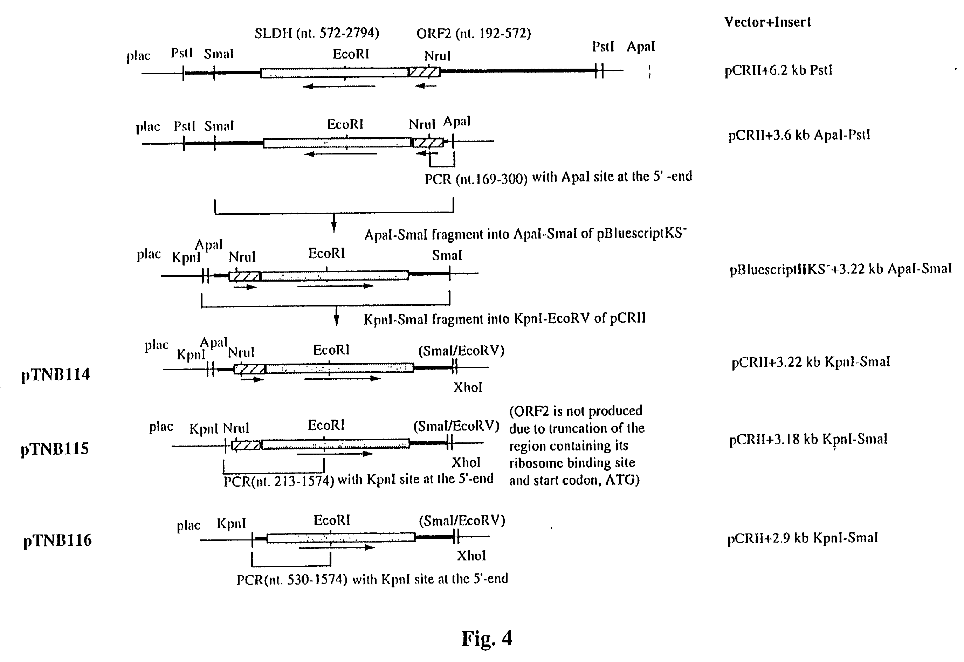

Figure 4 illustrates the steps for constructing plasmids pTNB114, pTNB115, and pTNB116 for the expression of SLDH and/or ORF2 genes in *E*. *coli* under the control of lac promoter.

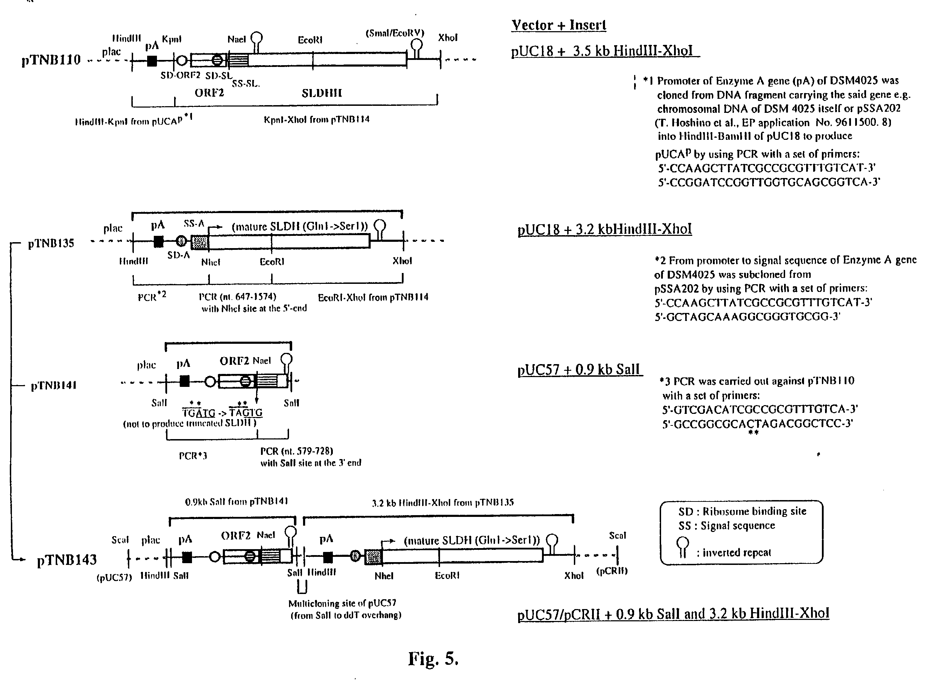

Figure 5 illustrates the steps for constructing plasmids pTNB110 which carries ORF2 and SLDH genes under the control of common pA promoter (described below in Example 6) and pTNB143 which carries ORF2 gene under the control of pA located adjacent to the gene in the upstream and SLDH gene under the control of another pA promoter located adjacent to the SLDH gene in its upstream.

The inventors have isolated SLDH gene together with a gene functionable in developing SLDH activity in vivo by DNA recombinant techniques and determined the nucleotide sequences.

The present invention provides DNA sequences encoding *Gluconobacter* SLDH and a gene functionable in developing SLDH activity in vivo herein after referred to as ORF2 gene, expression vectors containing the said DNA for SLDH and host cells carrying the said expression vectors.

Briefly, the SLDH and/or ORF2 gene(s), the DNA molecule containing the said gene(s), the recombinant expression vector and the recombinant organism utilized in the present invention can be obtained by the following steps:
(1) Isolating chromosomal DNA from the microorganisms which can provide SLDH activity that converts D-sorbitol to L-sorbose and constructing the gene library with the chromosomal DNA in an appropriate host cell, e. g. *E*. *coli*.
(2) Cloning SLDH and/or ORF2 gene(s) from a chromosomal DNA by colony-, plaque-, or Southern-hybridization, PCR (polymerase chain reaction) cloning, Western-blot analysis and the like.
(3) Determining the nucleotide sequence of the SLDH and/or ORF2 gene(s) obtained as above by conventional methods to select DNA molecule containing said SLDH and/or ORF2 gene(s) and constructing the recombinant expression vector on which SLDH and/or ORF2 gene(s) can express efficiently.
(4) Constructing recombinant organisms carrying SLDH and/or ORF2 gene(s) by an appropriate method for introducing DNA into host cell, e. g. transformation, transduction, transconjugation and/or electroporation.

The materials and the techniques used in the above aspect of the present invention are exemplified in details as follows:
A total chromosomal DNA can be purified by a procedure well known in the art. The aimed gene can be cloned in either plasmid or phage vectors from a total chromosomal DNA typically by either of the following illustrative methods:
   (i) The partial amino acid sequences are determined from the purified proteins or peptide fragments thereof. Such whole protein or peptide fragments can be prepared by the isolation of such a whole protein or by peptidase-treatment from the gel after SDS-polyacrylamide gel electrophoresis. Thus obtained protein or fragments thereof are applied to protein sequencer such as Applied Biosystems automatic gas-phase sequencer 470A. The amino acid sequences can be utilized to design and prepare oligonucleotide probes and/or primers with DNA synthesizer such as Applied Biosystems automatic DNA sequencer 381A. The said probes can be used for isolating clones carrying the objective gene from a gene library of the strain carrying the objective gene with the aid of Southern-, colony- or plaque-hybridization.
   (ii) Alternatively, for the purpose of selecting clones expressing aimed protein from the gene library, immunological methods with antibody prepared against the aimed protein can be applied.
   (iii) The DNA fragment of the aimed gene can be amplified from the total chromosomal DNA by PCR method with a set of primers, i.e. two oligonucleotides synthesized according to the amino acid sequences determined as above. Then a clone carrying the aimed-whole gene can be isolated from the gene library constructed, e.g. in *E*. *coli* by Southern-, colony-, or plaque-hybridization with the PCR product obtained above as the probe.
   (iv) A further alternative way of the cloning is screening of the clone complementing SLDH-deficient strain constructed by conventional mutation with chemical mutagenesis or by recombinant DNA techniques e.g. with transposon Tn5 to disrupt aimed gene.

DNA sequences which can be made by the polymerase chain reaction by using primers designed on the basis of the DNA sequences disclosed therein by methods known in the art are also an object of the present invention. It is understood that the DNA sequences of the present invention can also be made synthetically as described, e.g. in EP 747 483.

Above mentioned antibody can be prepared with purified SLDH protein or its peptide fragment as an antigen by such method described in Methods in Enzymology, vol. 73, p 46, 1981.

Once a clone carrying the desired gene is obtained, the nucleotide sequence of the aimed gene can be determined by a well known method such as dideoxy chain termination method with M13 phage (Sanger F. et al., Proc. Natl. Acad. Sci. USA, 74:5463-5467, 1977).

The desired gene expressing the D-sorbitol dehydrogenase activity of the present invention is illustrated in Fig. 2 and Fig. 3. This specific gene encodes the SLDH enzyme having 716 amino acid residues together with a signal peptide of 24 amino acid residues. The inventors found an open reading frame just upstream of the above SLDH structure gene and designated it as ORF2. This ORF2 gene encodes a protein having 126 amino acid residues, and was suggested to be functional in providing the desired enzymatic activity to the recombinant SLDH of the present invention, in particular when the said SLDH is expressed in a host cell of a different genus from acetic acid bacteria.

To express the desired gene/nucleotide sequence isolated from acetic acid bacteria including genus *Gluconobacter* and genus *Acetobacter* efficiently, various promoters can be used; for example, the original promoter of the gene, promoters of antibiotic resistance genes such as kanamycin resistant gene of Tn5 (D. E. Berg, and C. M. Berg. 1983. Bio/Technology 1:417-435), ampicillin resistant gene of pBR322, and β-galactosidase of *E*. *coli* (lac), trp-, tac-, trc-promoter, promoters of lambda phage and any promoters which can be functional in a host organism. For this purpose, the host organism can be selected from microorganisms including bacteria such as *Escherichia coli*, *Pseudomonas putida*, *Acetobacter xylinum*, *Acetobacter pasteurianus*, *Acetobacter aceti*, *Acetobacter hansenii*, and *Gluconobacter albidus*, *Gluconobacter capsulatus*, *Gluconobacter cerinus*, *Gluconobacter dioxyacetonicus*, *Gluconobacter gluconicus*, *Gluconobacter industrius*, *Gluconobacter melanogenus*, *Gluconobacter nonoxygluconicus*, *Gluconobacter oxydans*, *e*.*g*. *Gluconobacter oxydans DSM 4025, which had been deposited as DSM 4025 on March 17, 1987 under the conditions of the Budapest Treaty at the Deutsche Sammlung von Mikroorganismen und Zellkulturen GmbH*, *Braunschweig, BRD*, *Gluconobacter oxydans subsp*. *sphaericus*, *Gluconobacter roseus*, *Gluconobacter rubiginosus*, *Gluconobacter suboxydans*, mammalian cells and plant cells.

For expression, other regulatory elements, such as a Shine-Dalgarno (SD) sequence (for example, AGGAGG etc. including natural and synthetic sequences operable in the host cell) and a transcriptional terminator (inverted repeat structure including any natural and synthetic sequence operable in the host cell) which are operable in the host cell into which the coding sequence will be introduced can be used with the above described promoters.

For the expression of membrane-bound polypeptides, like the SLDH protein of the present invention, a signal peptide, which contains usually 15 to 50 amino acid residues and is totally hydrophobic, is preferably associated. A DNA encoding a signal peptide can be selected from any natural and synthetic sequence operable in the desired host cell.

A wide variety of host/cloning vector combinations may be employed in cloning the double-stranded DNA. The cloning vector is generally a plasmid or phage which contains a replication origin, regulatory elements, a cloning site including a multi-cloning site and selection markers such as antibiotic resistance genes including resistance genes for ampicillin, tetracycline, kanamycin, streptomycin, gentamicin, spectinomycin etc.

Preferred vectors for the expression of the gene of the present invention in *E*. *coli* is selected from any vectors usually used in *E*. *coli*, such as pBR322 or its derivatives including pUC18 and pBluescript II (Stratagene Cloning Systems, CA, USA), pACYC177 and pACYC184 (J. Bacteriol., 134:1141-1156, 1978) and their derivatives, and a vector derived from a broad host range plasmid such as RK2 (C. M. Thomas, Plasmid 5: 10, 1981) and RSF1010 (P. Guerry et al., J. Bacteriol. 117: 619-630, 1974). A preferred vector for the expression of the nucleotide sequence of the present invention in bacteria including *Gluconobacter*, *Acetobacter* and *P*. *putida* is selected from any vectors which can replicate in *Gluconobacter*, *Acetobacter* and/or *P*. *putida*, as well as in a preferred cloning organism such as *E*. *coli*. The preferred vector is a broad-host-range vector such as a cosmid vector like pVK100 (V. C. Knauf et al., Plasmid 8: 45-54, 1982) and its derivatives and RSF1010. Copy number and stability of the vector should be carefully considered for stable and efficient expression of the cloned gene and also for efficient cultivation of the host cell carrying the cloned gene. DNA molecules containing transposable elements such as Tn5 can be also used as a vector to introduce the desired gene into the preferred host, especially on a chromosome. DNA molecules containing any DNAs isolated from the preferred host together with the gene of the present invention is also useful to introduce this gene into the preferred host, especially on a chromosome. Such DNA molecules can be transferred to the preferred host by applying any of a conventional method, e.g. transformation, transduction, transconjugation or electroporation, which are well known to those skilled in the art, considering the nature of the host and the DNA molecule.

Useful hosts may include microorganisms, mammalian cells, and plant cells and the like. As a preferable microorganism, there may be mentioned bacteria such as *E*. *coli*, *P*. *putida*, *A*. *xylinum*, *A*. *pasteurianus*, *A*. *aceti*, *A*. *hansenii*, *Gluconobacter albidus*, *Gluconobacter capsulatus*, *Gluconobacter cerinus*, *Gluconobacter dioxyacetonicus*, *Gluconobacter gluconicus*, *Gluconobacter industrius*, *Gluconobacter melanogenus*, *Gluconobacter nonoxygluconicus*, *Gluconobacter oxydans*, *Gluconobacter oxydans subsp*. *sphaericus*, *Gluconobacter roseus*, *Gluconobacter rubiginosus*, *Gluconobacter suboxydans*, and any bacteria which are capable of expressing recombinant SLDH and/or ORF2 gene(s). Functional equivalents, subcultures, mutants and variants of said microorganism can be also used in the present invention. A preferred strain is *E*. *coli* K12 or its derivatives, *P*. *putida*, *Gluconobacter*, or *Acetobacter* strains.

The SLDH and/or ORF2 gene(s)/nucleotide sequences provided in this invention are ligated into a suitable vector containing a regulatory region such as a promoter, a ribosomal binding site and a transcriptional terminator operable in the host cell described above with a well-known methods in the art to produce an expression vector. When the SLDH and ORF2 genes are cloned in combination, the two genes can be cloned either in tandem or separately on the same plasmid and also on the chromosomal DNA. Figures 4 and 5 exemplifies the form of the combined cloning of SLDH and ORF2 genes on the plasmid. One may clone also one gene on a plasmid and the other one on chromosomal DNA.

To construct a recombinant microorganism carrying a recombinant expression vector, various gene transfer methods including transformation, transduction, conjugal mating (Chapters 14 and 15, Methods for general and molecular bacteriology, Philipp Gerhardt et al. ed., American Society for Microbiology, 1994), and electroporation can be used. The method for constructing a recombinant organism may be selected from the methods well-known in the field of molecular biology. Usual transformation systems can be used for *E*. *coli*, *Pseudomonas*, *Gluconobacter* and *Acetobacter*. A transduction system can also be used for *E*. *coli*. Conjugal mating system can be widely used in Gram-positive and Gram-negative bacteria including *E*. *coli*, *P. putida* and *Gluconobacter*. A preferred conjugal mating is disclosed in WO89/06688. The conjugation can occur in liquid medium or on a solid surface. The preferred recipient for SLDH and/or ORF2 production is selected from *E. coli, P. putida*, *Gluconobacter* and *Acetobacter*. To the recipient for conjugal mating, a selective marker is usually added; for example, resistance against nalidixic acid or rifampicin is usually selected. Natural resistance can be also used; e.g. resistance against polymyxin B is useful for many *Gluconobacters*.

The present invention provides a process for producing recombinant SLDH. One can increase the production yield of the SLDH enzyme by introducing the gene of SLDH provided by the present invention into organisms including acetic acid bacteria including the genus *Gluconobacter* and the genus *Acetobacter*. One can also produce active SLDH in microorganisms beside *Gluconobacter* by using the SLDH gene of the present invention in combination with the ORF2 gene of the present invention. The recombinant SLDH can be immobilized on a solid carrier for solid phase enzyme reaction. The present invention also provides recombinant organisms. One can produce L-sorbose from D-sorbitol with the recombinant organisms. One can also produce L-sorbose from D-sorbitol even in a host organism beside acetic acid bacteria by introducing the SLDH gene in combination with the ORF2 gene of the present invention.

### Examples

### Example 1.Determination of amino acid sequences of SLDH

### (1) N-terminal amino acid sequence analyses of lysylendopeptidase-treated SLDH polypeptides

Partial amino acid sequences of peptides No.1, 3 and 8 prepared from SLDH protein were determined (Fig. 1; SEQ ID NO:4 to 6). Purified SLDH of *G. suboxydans* IFO 3255 (T. Hoshino et al., EP 728840) was digested with lysylendopeptidase; the reaction mixture (25 ml) contained 1.2 mM of lysylendopeptidase and 3 nmol of the SLDH protein in 100 mM Tris-HCl buffer, pH9.0, and the reaction was carried out at 37°C for 15 hours. The resulting peptide fragments were separated by HPLC (column : protein C-4, VYDAC, California, USA) with acetonitrile/isopropanol gradient in 0.1% TFA at a flow rate of 1.0 ml/min. Elution of the peptides was monitored by UV absorbance at 214 nm, and the peak fractions were collected manually and subjected to N-terminal amino acid sequence analysis with an amino acid sequencer (Applied Biosystems model 470A, The Perkin Elmer Corp., Conn., USA).

### Example 2. Cloning of partial SLDH gene by PCR

PCR was conducted with chromosomal DNA of *G. suboxydans* IFO 3255 and degenerate oligonucleotide DNA primers, s7 and s6R whose sequences are shown in Fig. 1. The PCR amplification was carried out with thermostable polymerase Amplitaq (Perkin-Elmer, Roche Molecular Systems Inc., NJ, USA), using a thermal cycler (ZYMOREACTOR II AB-1820, ATTO, Tokyo, Japan). The following reaction mixture (50 µl) was used for PCR: 200 µM of dNTPs, 10 - 20 pmol of each primer (54 - 288 degeneracy), 6 ng of chromosomal DNA of *G. suboxydans* IFO 3255 and 0.5 unit of the DNA polymerase in the buffer provided from the supplier. The reaction consisted of 30 cycles of 1) denaturation step at 48°C for 1 min; 2) annealing step at 94°C for 1 min; 3) synthesis step at 72°C for 2 min. Consequently, 1.6 kb DNA was amplified and cloned in *E. coli* vector, pUC57/T (MBI Fermentas, Vilnius, Lithuania), which has 3'-ddT-tailed ends for direct ligation of an amplified DNA fragment to obtain a recombinant plasmid pMT20. The cloned DNA was subjected to nucleotide sequencing by the method of dideoxy-chain termination (F. Sanger et al, Proc. Natl. Acad. Sci. USA, 74:5463-5467, 1977); the 1.6 kb fragment encoded the peptides No. 3 and No. 8.

### Example 3. Complete cloning of the SLDH gene

### (1) Construction of gene library of G. suboxydans IFO 3255

The chromosomal DNA of *G*. *suboxydans* IFO 3255 was prepared from the cells grown on MB agar plate for 2 days. The chromosomal DNA (160 µg) was partially digested with 20 units of *Eco* RI in 500 µl of reaction mixture. Portions of the sample were withdrawn at 5, 10, 15, 30, and 60 minutes and the degree of the digestion was detected by agarose gel electrophoresis. Former four portions which contained partially-digested DNA fragments were combined and subjected to preparative gel electrophoresis (agarose: 0.6%). Fragments of 15 - 35 kb were cut out and electroeluted from the gel. The eluate was filtered and precipitated with sodium acetate and ethanol at -80°C. The DNA fragments were collected by centrifugation and suspended in 200 µl of 10 mM Tris-HCl, pH8.0, buffer containing 1 mM EDTA.

In parallel, 1.8 µg of a cosmid vector pVK100 was completely digested with *Eco* RI and treated with calf intestine alkaline phosphatase. The linearized and dephosphorylated pVK100 was ligated with 15 - 35 kb *Eco* RI fragments of the chromosomal DNA of *G*. *suboxydans* IFO 3255 (5 µg) with the ligation kit (Takara Shuzo, Kyoto, Japan) in 20 separate tubes under the condition recommended by the supplier to obtain highly polymerized DNA. The ligated DNA was then used for in vitro packaging according to the method described by the supplier (Amersham Japan). The resulting phage particles were used to infect *E. coli* ED8767, a host for the genomic library. Consequently, 4,271 colonies were obtained and all of the colonies tested (20 colonies) possessed the insert DNAs with the average size of about 25 kb.

### (2) Colony hybridization to obtain complete SLDH gene

The cosmid library described above was screened to isolate the clone carrying complete SLDH gene by colony hybridization method with ³²P-labeled 1.6 kb DNA of pMT20. One clone was isolated and designated pSLII, which carry about 25 kb insert in pVK100 vector. From the pSLII, 6.2 kb *Pst* I fragment was isolated and cloned into pUC18 to obtain plasmid pUSLIIP. The 6.2 kb *Pst* I fragment containing ORF2 and SLDH genes was isolated from pUSLIIP and subcloned into pCRII vector (Invitrogen Corporation, CA, USA) to produce pCRSLIIP. From the resulting plasmid, the DNA fragment containing the 6.2 kb *Pst* I fragment was isolated as *Hind* III-*Xho* I fragment and cloned between *Hind* III and *Xho* I sites of pVK100 to obtain pVKSLIIP.

### (3) Expression of SLDH gene in E. coli

To confirm whether 6.2 kb *Pst* I-fragment encodes the aimed SLDH, cells of *E. coli* JM109 carrying pUSLIIP was subjected to Western-blot analysis with anti-SLDH antibody as described above. Immuno-positive proteins with a molecular weight of about 80 kDa were observed in the transformant, indicating that the *Pst* I-fragment encodes the polypeptides with the molecular weight of the intact SLDH (79 kDa +/- 0.5 kDa).

### (4) Construction of SLDH-deficient Gluconobacter, strain 26A11, as the test strain for SLDH-activity complementation

Transposon Tn5 mutagenesis was performed with *G. melanogenus* IFO 3293 as the parent. Tn5, a transposable element coding for Kmr, causes null mutations at random on DNA of its host organism and widely used as a mutagen in Gram-negative bacteria. The IFO 3293 was selected as the parent in the following reasons: (i) it produced L-sorbose from D-sorbitol, (ii) it showed immuno-positive polypeptide of about 80 kDa in Western-blot analysis with the antibody prepared against SLDH purified from *G. suboxydans* IFO 3255 and (iii) its frequency for generating Tn5 mutants was much higher than that of *G. suboxydans* IFO 3255.

*G. melanogenus* IFO 3293 was cultivated in a test tube containing 5 ml of the MB medium containing 25 g/l of mannitol, 5 g/l of yeast extract (Difco Laboratories, ), 3 g/l of Bactopepton (Difco) at 30°C overnight. *E. coli* HB101 (pRK2013) [D. H. Figurski, Proc, Natl. Acad. Sci. USA 76: 1648 - 1652, 1979] and *E. coli* HB101 (pSUP2021) [R. Simon, et al., BIO/TECHNOL. 1:784-791, 1983] were cultivated in test tubes containing 5 ml of LB medium with 50 µg/ml of kanamycin at 37°C overnight. The cells was separately collected by centrifugation and suspended in the half volume of MB medium. The each cell suspension was mixed in the ratio of 1:1:1 and the mixture was placed on the nitrocellulose filter on the surface of MB agar plate. The plate was incubated at 27°C overnight and the resulting cells on the filter was scraped, suspended in the appropriate volume of MB medium and spread on the selection plate (MPK plate), MB containing 10 µg/ml of polymyxin B and 50 µg/ml of kanamycin. The MPK plate was incubated at 27°C for 3 to 4 days.

The resulting 3,436 Tn5 mutants were subjected to the immuno-dot blot screening with the anti-SLDH antibody. The cells of each strain were independently suspended in 50 µl Laemmli buffer consisting of 62.5 mM Tris-HCl (pH6.5), 10% glycerol, 2% SDS and 5% β-mercaptoethanol in 96-well microtiter plate and incubated at 60°C for 2 hours. The Cell Free Extracts (CFEs) were stamped on the nitrocellulose filter and the immuno-positive samples in the CFEs were screened with AP conjugate substrate kit (Bio-RAD Laboratories, Richmond, Calif., USA). As a result, only one strain 26A11 without positive signal in the immuno-dot blot screening was obtained.

SLDH-deficiency of the strain 26A11 was confirmed by Western-blot analysis; it expressed at most 1/500 amount of SLDH compared with its parent strain. 26A11 was not a complete SLDH-deficient strain but the strain with SLDH gene repressed by Tn5 insertion; the insertion site was found to be close to the C-terminus by determining the nucleotide sequence around Tn5-insertion point. Next, a resting cell reaction was conducted to examine the whole SLDH activity in 26A11 and the wild Gluconobacter strains. In the potassium phosphate buffer 100 mM (pH7.0) containing 2% D-sorbitol, 26A11 slightly converted D-sorbitol to L-sorbose, whereas the wild strains IFO 3293 and IFO 3255 completely did it in 39.5 hr at 30°C.

### (5) Expression of SLDH gene in 26A11

To confirm the SLDH activity of the SLDH clones obtained, complementation test was conducted. Plasmids pSLII and pVKSLIIP were introduced into 26A11 by a conjugal mating. The transconjugant carrying pSLII or pVKSLIIP restored the activity of SLDH in a mini-resting cell reaction and showed immuno-reactive polypeptide of about 80 kDa in Western-blot analysis.

### (6) Nucleotide sequencing of the SLDH gene

Plasmid pUSLIIP was used for nucleotide sequencing of SLDH and ORF2 genes. Determined nucleotide sequence (SEQ ID NO: 1; 3,481 bp) revealed that ORF of SLDH gene (2,223 bp, nucleotide No. 572 to 2794 in SEQ ID NO: 1) encoded the polypeptide of 740 amino acid residues (SEQ ID NO: 2), in which there were three amino acid sequences (Peptides No. 1, 3, and 8 shown in SEQ ID NO: 4 to 6) determined from the purified SLDH polypeptide. In addition to the SLDH ORF, one more ORF, ORF2, was found just upstream of SLDH ORF as illustrated in Figs. 2 and 3. The ORF of ORF2 (381 bp, nucleotide No. 192 to 572 in SEQ ID NO: 1) encoded the polypeptide of 126 amino acid residues (SEQ ID NO. 3).

The 4^{th} amino acid sequence of Peptide No.1 was determined as Glu by the amino acid sequencer, but it was Ala according to the DNA sequence. Thell^{th} amino acid sequence of Peptide No.3 was determined as Gln by the amino acid sequencer, but it was Pro according to the DNA sequence. A signal peptide-like region (SEQ ID NO: 8) is possibly included in the deduced amino acid sequence: it contains (i) many hydrophobic residues, (ii) a positively-charged residues near N-terminus, and (iii) Ala-Xaa-Ala site as a cleaved signal. The actual signal sequence was determined as described in Example 3 (7). The putative ribosome-binding site (Shine-Dalgarno, SD, sequence) for SLDH gene was located at 8 bp upstream of the initiation codon (AGAGGAG at nucleotide No. 558 - 564 of Seq ID NO: 1). The putative SD sequence for ORF2 gene was located at 10 bp upstream of the initiation codon (GGGAGG at nucleotide No. 177 to 182 of Seq ID NO: 1). There were some inverted repeat sequences immediately downstream the SLDH structure gene (nucleotide sequences of No. 2803 - 2833 and of No. 2838 - 2892) as illustrated in Fig. 3; they may function as transcription termination loops for SLDH gene. For ORF2 gene, the inverted repeat sequence was found at No. 684 - 704.

Homology search for SLDH and ORF2 was performed with the programs of mpBlast (NCBI, Bethesda, Md. USA) and Motifs in GCG (Genetics Computer Group, University Research Park, WI, USA). SLDH polypeptide had the sequence commonly conserved in quinoprotein at the region near C-terminus (within amino acid residue No. 632 to 692 of SEQ ID No. 2) and the other sequence identified as a quinoprotein motifs (Prosite No. PS00363: [DN]W.{3}G[RK].{6}[FY]S.{4}[LIVM]N.{2}NV.{2}L[RK]; amino acid residue No. 79 to 107 of SEQ ID No. 2).

ORF2 showed homology with the N-terminal region of the membrane-bound PQQ-dependent D-glucose dehydrogenase (GDH) of *G. oxydans*, *E. coli*, *Acinetobacter calcoaceticus*, which is known as a membrane-spanning region to bind the GDH to the membrane. Identities of ORF2 to the N-terminal region of the GDHs of *G. oxydans*, *E. coli, A. calcoaceticus* were 30%, 32%, and 37%, respectively. The ORF2 protein may function as an anchoring protein to make the SLDH membrane-bound type.

### Example 4. Determination of N-terminal and C-terminal sequences of mature SLDH polypeptide

Direct sequencing of the N-terminus gave no results, indicating that the N-terminus is blocked. Then, SLDH polypeptide was treated with the endoproteinase Lys-C (Wako, Osaka Japan ) in 0.1 M Tris-HCl at 37°C for 20 hours with a substrate-to-enzyme ratio of 20:1 (w/w). Total digest was analyzed by reversed phase HPLC (RP300, 1 mm x 25 cm, Applied Biosystems, Foster City, CA) and each peak was subjected to mass spectrometer (TSQ700 triple quadrupole instrument, Finnigan-MAT, San Jose, California) for determining molecular weight. One of the digest described as SEQ ID No. 7 was assigned as the N-terminal sequence by the mass spectrometric analysis and amino acid composition analysis together with the amino acid composition predicted from determined nucleotide sequence shown as SEQ ID NO: 1. Further analysis with collisional induced dissociation (CID) was carried out to confirm the identity of the peptide with N-terminal sequence. The N-terminus was determined to be pyroglutamyl residue.

Since the N-terminus of SLDH was determined to be Gln-Phe-Ala-Pro-Ala-Gly-Ala-Gly-Gly-Glu-Pro-Ser-Ser-Ser-Val-Pro-Gly-Pro-Gly-Asn-Ala-Ser-Glu-Pro-Thr-Glu-Asn-Ser-Pro-Lys as shown in SEQ ID NO. 7, the signal sequence was confirmed to be 24 amino acid residue long with the sequence of Met-Arg-Arg-Pro-Tyr-Leu-Leu-Ala-Thr-Ala-Ala-Gly-Leu-Ala-Leu-Ala-Cys-Ser-Pro-Leu-Ile-Ala-His-Ala as listed as SEQ ID NO: 8. The C-terminal sequence was also determined by using the peptide recovered from V8 protease digest to be Pro-Asp-Ala-Ile-Lys-Gln (SEQ ID NO: 9).

### Example 5. Expression of the SLDH and/or ORF2 gene(s) in E. coli

From the pCRSLIIP described in Example 3 (2), plasmids carrying SLDH gene with or without ORF2 gene under the lac promoter control were constructed as illustrated in Fig. 4. The resulting three plasmids are pTNB114 carrying SLDH and ORF2 genes, pTNB115 carrying SLDH gene and ORF2 gene truncated at its N-terminus containing ribosome binding site and start codon (ATG) and pTNB116 carrying mostly-truncated ORF2 gene and intact SLDH gene. These three plasmids were introduced into *E. coli* by conventional transformation. The production of the SLDH polypeptide was detected by Western-blot analysis with cell free extracts of the resulting transformants and the SLDH activity was assayed with resting cells. The resting cell reaction was carried out in the reaction mixture consisting of 0.3% NaCl, 1% CaCO₃, 4% D-sorbitol, and 1 mM PMS with or without 1 µg/ml of PQQ at room temperature for 17 hours. The SLDH activity to produce L-sorbose was analyzed by TLC assay with Silica gel 60 F₂₅₄, 0.25 mm, Merck with the developing solvent consisting of *n*-propanol-H₂O-1% H₃PO₄-HCOOH (400:100:10:1) and spray reagent of naphthoresorcinol. Consequently, SLDH polypeptide was detected in all transformants, even without ORF2 gene expression, in Western-blot analysis, but SLDH activity to produce L-sorbose was detected only in transformant carrying pTNB114 containing intact ORF2 gene under the resting cell reaction condition in the presence of PQQ.

### Example 6. Expression of the SLDH and/or ORF2 gene(s) in E. coli

Figure 5 illustrates construction steps of pTNB110 and pTNB143. Plasmid pTNB110 carrying ORF2 and SLDH genes under control of the promoter of Enzyme A gene (pA) of DSM4025 (T. Hoshino et al., European Patent Application No. 9611500.8) was constructed by inserting *Hind* III-*Kpn* I fragment containing pA, *Kpn* I-*Xho* I fragment from pTNB114 between *Hind* III-*Xho* I of pUC18. Plasmid pTNB143 carrying ORF2 and SLDH genes as independent expression units, ORF2 gene with pA and SLDH gene with pA, was constructed by inserting 0.9 kb *Sal* I fragment from pTNB141 and 3.2 kb *Hind* III-*Xho* I fragment from pTNB135 into pUC57/pCRII hybrid vector (*Sca* I-*Hind* III fragment with plac from pUC57 and *Xho* I to *Sca* I fragment without plac from pCRII) as shown in Fig. 5. The plasmids, pTNB110 and pTNB143, were introduced into *E. coli* by conventional transformation. The resulting transformants were subjected to Western-blot analysis and resting cell reaction as described in Example 5. Consequently, both of the transformants carrying pTNB110 and pTNB143 produced SLDH protein and showed SLDH activity to produce L-sorbose from D-sorbitol in the presence of PQQ.

### Example 7. Expression of the SLDH gene in G. oxydans DSM 4025

The plasmid pTNB136 for the expression of SLDH gene in *G. oxydans* DSM 4025 having strong L-sorbose and L-sorbosone dehydrogenase activities together with weak D-sorbitol dehydrogenase activity to produce L-sorbose, was constructed by inserting *Hind* III-*Xho* I fragment from pTNB135 (Fig. 5) between *Hind* III and *Xho* I sites of pVK100. The plasmid pTNB136 and its vector pVK100 were introduced by conjugal mating into strain GOBΔK, which is a mutant of *G. oxydans* DSM 4025 whose gene of Enzyme B (EP 832 974) having D-sorbitol dehydrogenase to produce L-sorbose is deleted by replacing two *Eco* RI fragments containing Enzyme B gene with kanamycin resistant gene cassette (1.28 kb *Eco* RI fragment of pUC4K; Pharmacia Uppsala, Sweden). The gene disruption was conducted by the recombinant DNA techniques well-known in the art. The resulting transconjugant, GOBΔK carrying pTNB136 or pVK100, produced 5 g/L or below 2 g/l of 2KGA in 10% D-sorbitol, respectively, by flask fermentation conducted at 30°C for 4 days (medium: 10% D-sorbitol, 1.6% urea, 0.05% glycerol, 0.25% MgSO₄•7H₂O, 3% corn steep liquor, 6.25% baker's yeast wet cells and 1.5% CaCO₃). Western-blot analysis with anti-SLDH antibody revealed that the transconjugants carrying pTNB136 expressed the immuno-reactive SLDH polypeptides.

### SEQUENCE LISTING

(1) GENERAL INFORMATION
(i) APPLICANT

| | |
|---|---|
| NAME: | F. HOFFMANN-LA ROCHE AG |
| | |
| STREET: | Grezacherstrasse 124 |
| | |
| CITY: | Basle |
| | |
| COUNTRY: | Switzerland |
| | |
| POSTAL CODE: | CH-4002 |
| | |
| TELEPHONE: | 061 - 688 25 11 |
| | |
| FAX: | 061 - 688 13 95 |
| | |
| TELEX: | 962292/965542 hlr c |

(ii) TITLE OF INVENTION:
D-Sorbitol dehydrogenase gene

(iii) NUMBER OF SEQUENCES: 9
(iv) COMPUTER READABLE FORM:
(A) MEDIUM TYPE: Floppy disk
(B) COMPUTER: Macintosh
(C) OPERATING SYSTEM:
(D) SOFTWARE: MS word ver 5.1a

(v) CURRENT APPLICATION DATA:
(A) APPLICATION NUMBER:
(B) FILING DATE:
(C) CLASSIFICATION

(2) INFORMATION FOR SEQ ID NO:1:
(i) SEQUENCE CHARACTERISTICS:

| | | |
|---|---|---|
| (A) | LENGTH: | 3481 base pairs |
| | | |
| (B) | TYPE: | nucleic acid |
| | | |
| (C) | STRANDEDNESS: | double |
| | | |
| (D) | TOPOLOGY: | linear |

(ii) MOLECULE TYPE: DNA (genomic)
(iii) ORIGINAL SOURCE:

| | |
|---|---|
| ORGANISM: | *Gluconobacter suboxydans* |
| | |
| STRAIN: | IFO 3255 |

(iv) FEATURE:

| | |
|---|---|
| FEATURE KEY: | CDS |
| | |
| POSITION: | 192..572 |
| | |
| SEQUENCING METHOD: | E |
| | |
| FEATURE KEY: | CDS |
| | |
| POSITION: | 572..2794 |
| | |
| SEQUENCING METHOD: | E |

ACAAATCATA CTGGCGGCGC TGTAGTGACA ATTCCGGCGG GTTAAAGAGA ATATTTTTTT 60

GGTGACAGGC CACAACAAAT TTTTGTTACC TCAAACACAG TTTTGTTAGA GCATTTGAAA 120

ACGAAGTCCG ATGGACCTGA ACTGAATATG GATTTACCGT CCGGAGGATT CAGTTTGGGA 180

GGCATTCGGT TATGCCAAAT CTTCAAGGTA ATAGGACTCT GACGGAGTGG CTGACGCTGC 240

TTCTCGGGGT CATCGTCCTT CTTGTGGGCC TGTTCTTCGT CATTGGGGGT GCTGACCTCG 300

CGATGCTGGG CGGCTCTACC TACTATGTTC TCTGTGGCAT CCTCCTGGTT GCTAGCGGCG 360

TATTCATGCT CATGGGCCGC ACGCTTGGTG CCTTCCTGTA TCTGGGTGCC CTGGCCTACA 420

CGTGGGTCTG GTCCTTCTGG GAAGTCGGTT TCAGCCCCAT CGATCTTCTG CCCCGCGCTT 480

TCGGCCCGAC CATCCTTGGC ATTCTCGTTG CCCTGACCAT TCCGGTCCTG CGCCGCATGG 540

AAAGCCGTCG TACTCTCAGA GGAGCCGTCT GATGCGCCGG CCTTACCTTC TAGCAACAGC 600

CGCAGGACTC GCCCTTGCCT GTTCGCCGCT CATCGCTCAT GCACAGTTTG CTCCCGCAGG 660

GGCTGGCGGC GAACCTTCCT CGTCAGTTCC TGGGCCAGGA AATGCGAGCG AGCCCACCGA 720

AAACTCTCCG AAAAGTCAGA GCTACTTCGC AGGACCGTCG CCCTATGCCC CGCAGGCTCC 780

TGGCGTAAAC GCAGCCAACC TGCCGGACAT TGAGTCAATC GATCCCTCGC AGGTCCCGGC 840

CATGGCTCCG CAGCAGAGTG CCAATCCGGC ACGTGGAGAC TGGGTTGCTT ACGGACGTGA 900

CGATCATCAG ACGCGATACT CTCCGCTTTC GGAAATCACG CCTGAGAACG CAAGCAAGCT 960

CAAGGTCGCT TTCGTCTACC ACACGGGGAG TTATCCGCGT CCGGGACAGG TGAACAAATG 1020

GGCCGCCGAA ACCACGCCGA TCAAGGTTGG TGACGGTCTC TACACATGTT CCGCCATGAA 1080

CGACATCATC AAGCTGGATC CGGCTACGGG TAAGCAGATC TGGCGTCGGA ACGTGGATGT 1140

CAAATACCAC TCCATTCCCT ATACCGCTGC CTGTAAGGGT GTGACGTATT TCACGTCCTC 1200

CGTGGTGCCG GAAGGCCAGC CCTGCCACAA TCGCCTTATC GAAGGCACGC TGGATATGCG 1260

TCTGATTGCG GTTGACGCGG AGACAGGGGA TTTCTGCCCT AATTTCGGTC ATGGTGGTCA 1320

GGTCAACCTG ATGCAGGGTC TGGGTGAGTC TGTTCCGGGC TTCGTCTCCA TGACGGCACC 1380

TCCACCGGTC ATCAACGGCG TCGTGGTTGT AAACCACGAA GTGCTCGACG GTCAGCGCCG 1440

CTGGGCTCCG TCCGGTGTGA TCCGTGGTTA CGATGCTGAA AGTGGCAAAT TCGTATGGGC 1500

CTGGGACGTC AACAATTCCG GACGATCACA GCCAGCCTAC CGGGTAACCG TCATTACAGC 1560

CGTGGAACGC CGAATTCCTG GGCTACCTGA CAGGCGACAA CGAGGAGGGT CTCGTTTACG 1620

TCCCGACAGG AACTCTGCTG CTGACTATTA CAGCGCCCTG CGTAGTGATG CTGAAAACAA 1680

GGTGTCCTCC GCTGTTGTCG CCATTGACGT CAAGACGGGT TCTCCGCGCT GGGTCTTCCA 1740

GACGGCTCAT AAGGACGTCT GGGATTATGA CATCGGTTCA CAGGCGACCC TGATGGATAT 1800

GCCTGGCCCG GATGGCCAGA CGGTTCCTGC TCTCATCATG CCGACCAAGC GTGGCCAGAC 1860

GTTCGTGCTT GACCGTCGTA CCGGCAAGCC AATTCTGCCG GTTGAAGAAC GCCCAGCTCC 1920

GTCCCCTGGT GTTATTCCGG GTGACCCGCG TTCTCCGACG CAGCCATGGT CCGTCGGGAT 1980

GCCGGCCCTT CGCGTGCCGG ATCTGAAAGA GACAGACATG TGGGGTATGT CCCCCATCGA 2040

TCAGCTCTTC TGCCGTATCA AGTTCCGCCG TGCGAACTAT GTGGGTGAGT TCACACCACC 2100

GAGCGTTGAC AAGCCGTGGA TTGAATATCC GGGCTATAAC GGTGGCAGTG ACTGGGGCTC 2160

CATGTCCTAT GATCCGCAGT CCGGCATCCT GATTGCGAAC TGGAACATCA CACCGATGTA 2220

CGACCAGCTC GTAACCCGCA AGAAGGCAGA CTCCCTCGGC CTGATGCCGA TCGATGACCC 2280

CAACTTCAAG CCAGGTGGCG GTGGTGCCGA AGGTAACGGC GCCATGGACG GAACGCCTTA 2340

CGGTATCGTC GTGACACCGT TCTGGGATCA GTACACGGGC ATGATGTGCA ACCGTCCGCC 2400

CTACGGTATG ATCACAGCCA TCGACATGAA GCACGGCCAG AAGGTTCTGT GGCAGCATCC 2460

GCTCGGAACG GCTCGCGCCA ACGGTCCATG GGGTCTGCCA ACAGGTCTGC CATGGGAAAT 2520

CGGCACTCCG AACAATGGTG GTTCGGTTGT GACCGGTGGC GGTCTGATCT TCATCGGTGC 2580

GGCAACGGAT AACCAGATCC GCGCGATTGA TGAACACACT GGCAAGGTTG TCTGGAGCGC 2640

AGTCCTCCCC GGCGGCGGTC AGGCCAATCC GATGACGTAT GAAGCCAATG GTCACCAGTA 2700

CGTTGCCATC ATGGCTGGCG GTCATCACTT CATGATGACG CCAGTGTCTG ACCAGCTTGT 2760

GGTTTACGCA CTGCCGGATG CCATCAAGCA GTAATTAAGT CCTGTGGCGG ATGTGTCATG 2820

CATATCCGCC ACACTCCATC GTCAGAAGGA GACTTTCGTG CTAGCCATGC AGGGAAGTCT 2880

CCTTTTGACG TTTTTGGCTC TTTCCAGCGA GCGGGCAGTC TGAAACGGGG CTTCGTCTGG 2940

CTCGTACTTT CAGAATGGCT CGTCGCACCC TCATGACTGC CCACTCCCCC GTTATCTTGC 3000

AGGTTCTGCC AGCCCTCAGC ACGGGCGGCC TGGAGCGGGG AGCTATTGAA ATTGCGGCTG 3060

CCATCACACA GGCTGGTGGC AAGGCCATTG TCGCTTCGAA GACGGGTCCT CTTCTTGTGC 3120

AACTCCGCCA CGTCGGAGCA GTGCATGTGC CGCTGGATCT CAAATCGAAA TCGCCGTTTT 3180

CTGTTCGGCG CCGTGCCCGT GAACTCCAGA AACTGATCCG GGAGCAGCAG GTTGATCTGG 3240

TTCACGCCCG GTCCCGTATT CCGGCATGGG CCGCCTGGCT CGCCTGCCGC CGCGAGAACA 3300

TTCCTTTCGT GACAACGTGG CATGGCGTCC ACGAGGCTGG CTGGTGGGGC AAGAAATTCT 3360

ACAATTCGGT GCTGGCCCGG GGTGCAAGGG TCATCGCAAT TTCGCACTAC ATTTCCGGGC 3420

GTCTTTCAGG GCAGTACGGC GTTCAGGCAG ATCGTCTTCG AACCATTCCG CGTGGTGCCG 3480

A

INFORMATION FOR SEQ ID NO:2:
(i) SEQUENCE CHARACTERISTICS:

| | | |
|---|---|---|
| (A) | LENGTH: | 740 residues |
| | | |
| (B) | TYPE: | amino acid |
| | | |
| (C) | TOPOLOGY: | linear |

(ii) MOLECULE TYPE: protein
(iii) ORIGINAL SOURCE:

| | |
|---|---|
| ORGANISM: | *Gluconobacter suboxydans* |
| | |
| STRAIN: | IFO 3255 |

(iv) FEATURE:

| | |
|---|---|
| FEATURE KEY: | sig peptide |
| | |
| POSITION: | -24..-1 |
| | |
| SEQUENCING METHOD: | E |
| | |
| FEATURE KEY: | mat peptide |
| | |
| POSITION: | 1..716 |
| | |
| SEQUENCING METHOD: | E |

Met Arg Arg Pro Tyr Leu Leu Ala Thr Ala Ala Gly Leu Ala Leu
-24 -20 -15 -10
Ala Cys Ser Pro Leu Ile Ala His Ala Gln Phe Ala Pro Ala Gly

Ala Gly Gly Glu Pro Ser Ser Ser Val Pro Gly Pro Gly Asn Ala
10 15 20
Ser Glu Pro Thr Glu Asn Ser Pro Lys Ser Gln Ser Tyr Phe Ala
25 30 35
Gly Pro Ser Pro Tyr Ala Pro Gln Ala Pro Gly Val Asn Ala Ala
40 45 50
Asn Leu Pro Asp Ile Glu Ser Ile Asp Pro Ser Gln Val Pro Ala
55 60 65
Met Ala Pro Gln Gln Ser Ala Asn Pro Ala Arg Gly Asp Trp Val
70 75 80
Ala Tyr Gly Arg Asp Asp His Gln Thr Arg Tyr Ser Pro Leu Ser
85 90 95
Glu Ile Thr Pro Glu Asn Ala Ser Lys Leu Lys Val Ala Phe Val
100 105 110
Tyr His Thr Gly Ser Tyr Pro Arg Pro Gly Gln Val Asn Lys Trp
115 120 125
Ala Ala Glu Thr Thr Pro Ile Lys Val Gly Asp Gly Leu Tyr Thr
130 135 140
Cys Ser Ala Met Asn Asp Ile Ile Lys Leu Asp Pro Ala Thr Gly
145 150 155
Lys Gln Ile Trp Arg Arg Asn Val Asp Val Lys Tyr His Ser Ile
160 165 170

Pro Tyr Thr Ala Ala Cys Lys Gly Val Thr Tyr Phe Thr Ser Ser

175 180 185

Val Val Pro Glu Gly Gln Pro Cys His Asn Arg Leu Ile Glu Gly

190 195 200

Thr Leu Asp Met Arg Leu Ile Ala Val Asp Ala Glu Thr Gly Asp

205 210 215

Phe Cys Pro Asn Phe Gly His Gly Gly Gln Val Asn Leu Met Gln

220 225 230

Gly Leu Gly Glu Ser Val Pro Gly Phe Val Ser Met Thr Ala Pro

235 240 245

Pro Pro Val Ile Asn Gly Val Val Val Val Asn His Glu Val Leu

250 255 260

Asp Gly Gln Arg Arg Trp Ala Pro Ser Gly Val Ile Arg Gly Tyr

265 270 275

Asp Ala Glu Ser Gly Lys Phe Val Trp Ala Trp Asp Val Asn Asn

280 285 290

Ser Gly Arg Ser Gln Pro Ala Tyr Arg Val Thr Val Ile Thr Ala

295 300 305

Val Glu Arg Arg Ile Pro Gly Leu Pro Asp Arg Arg Gln Arg Gly

310 315 320

Gly Ser Arg Leu Arg Pro Asp Arg Asn Ser Ala Ala Asp Tyr Tyr

325 330 335

Ser Ala Leu Arg Ser Asp Ala Glu Asn Lys Val Ser Ser Ala Val

340 345 350

Val Ala Ile Asp Val Lys Thr Gly Ser Pro Arg Trp Val Phe Gln

355 360 365

Thr Ala His Lys Asp Val Trp Asp Tyr Asp Ile Gly Ser Gln Ala

370 375 380

Thr Leu Met Asp Met Pro Gly Pro Asp Gly Gln Thr Val Pro Ala

385 390 395

Leu Ile Met Pro Thr Lys Arg Gly Gln Thr Phe Val Leu Asp Arg

400 405 410

Arg Thr Gly Lys Pro Ile Leu Pro Val Glu Glu Arg Pro Ala Pro

415 420 425

Ser Pro Gly Val Ile Pro Gly Asp Pro Arg Ser Pro Thr Gln Pro

430 435 440

Trp Ser Val Gly Met Pro Ala Leu Arg Val Pro Asp Leu Lys Glu

D-Sorbitol dehydrogenase gene 445

450 455

Thr Asp Met Trp Gly Met Ser Pro Ile Asp Gln Leu Phe Cys Arg

460 465 470

Ile Lys Phe Arg Arg Ala Asn Tyr Val Gly Glu Phe Thr Pro Pro

475 480 485

Ser Val Asp Lys Pro Trp Ile Glu Tyr Pro Gly Tyr Asn Gly Gly

490 495 500

Ser Asp Trp Gly Ser Met Ser Tyr Asp Pro Gln Ser Gly Ile Leu
505 510 515
Ile Ala Asn Trp Asn Ile Thr Pro Met Tyr Asp Gln Leu Val Thr
520 525 530
Arg Lys Lys Ala Asp Ser Leu Gly Leu Met Pro Ile Asp Asp Pro
535 540 545
Asn Phe Lys Pro Gly Gly Gly Gly Ala Glu Gly Asn Gly Ala Met
550 555 560
Asp Gly Thr Pro Tyr Gly Ile Val Val Thr Pro Phe Trp Asp Gln
565 570 575
Tyr Thr Gly Met Met Cys Asn Arg Pro Pro Tyr Gly Met Ile Thr
580 585 590
Ala Ile Asp Met Lys His Gly Gln Lys Val Leu Trp Gln His Pro
595 600 605
Leu Gly Thr Ala Arg Ala Asn Gly Pro Trp Gly Leu Pro Thr Gly
610 615 620
Leu Pro Trp Glu Ile Gly Thr Pro Asn Asn Gly Gly Ser Val Val
625 630 635
Thr Gly Gly Gly Leu Ile Phe Ile Gly Ala Ala Thr Asp Asn Gln
640 645 650
Ile Arg Ala Ile Asp Glu His Thr Gly Lys Val Val Trp Ser Ala
655 660 665

Val Leu Pro Gly Gly Gly Gln Ala Asn Pro Met Thr Tyr Glu Ala

670 675 680

Asn Gly His Gln Tyr Val Ala Ile Met Ala Gly Gly His His Phe

685 690 695

Met Met Thr Pro Val Ser Asp Gln Leu Val Val Tyr Ala Leu Pro

700 705 710

Asp Ala Ile Lys Gln

715 716

INFORMATION FOR SEQ ID NO:3:
(i) SEQUENCE CHARACTERISTICS:

| | | |
|---|---|---|
| (A) | LENGTH: | 126 residues |
| | | |
| (B) | TYPE: | amino acid |
| | | |
| (C) | TOPOLOGY: | linear |

(ii) MOLECULE TYPE: protein
(iii) FRAGMENT TYPE: internal fragment
(iv) ORIGINAL SOURCE:

| | |
|---|---|
| ORGANISM: | *Gluconobacter suboxydans* |
| | |
| STRAIN: | IFO 3255 |

(v) FEATURE:

| | |
|---|---|
| FEATURE KEY: | mat peptide |
| | |
| POSITION: | 1..126 |
| | |
| SEQUENCING METHOD: | E |

Met Pro Asn Leu Gln Gly Asn Arg Thr Leu Thr Glu Trp Leu Thr
1               5                   10                  15
Leu Leu Leu Gly Val Ile Val Leu Leu Val Gly Leu Phe Phe Val
                20                  25                  30
Ile Gly Gly Ala Asp Leu Ala Met Leu Gly Gly Ser Thr Tyr Tyr
                35                  40                  45
Val Leu Cys Gly Ile Leu Leu Val Ala Ser Gly Val Phe Met Leu
                50                  55                  60

Met Gly Arg Thr Leu Gly Ala Phe Leu Tyr Leu Gly Ala Leu Ala
                65                  70                  75
Tyr Thr Trp Val Trp Ser Phe Trp Glu Val Gly Phe Ser Pro Ile
                80                  85                  90
Asp Leu Leu Pro Arg Ala Phe Gly Pro Thr Ile Leu Gly Ile Leu
                95                 100                 105
Val Ala Leu Thr Ile Pro Val Leu Arg Arg Met Glu Ser Arg Arg
               110                 115                 120
Thr Leu Arg Gly Ala Val
               125 126

INFORMATION FOR SEQ ID NO:4:
(i) SEQUENCE CHARACTERISTICS:

| | | |
|---|---|---|
| (A) | LENGTH: | 8 residues |
| | | |
| (B) | TYPE: | amino acid |
| | | |
| (C) | TOPOLOGY: | linear |

(ii) MOLECULE TYPE: peptide
(iii) FRAGMENT TYPE: internal fragment
(iv) ORIGINAL SOURCE:

| | |
|---|---|
| ORGANISM: | *Gluconobacter suboxydans* |
| | |
| STRAIN: | IFO 3255 |

(v) FEATURE:
SEQUENCING METHOD: E

Lys Trp Ala Glu Glu Thr Xaa Pro
1               5               8

INFORMATION FOR SEQ ID NO:5:
(i) SEQUENCE CHARACTERISTICS:

| | | |
|---|---|---|
| (A) | LENGTH: | 24 residues |
| | | |
| (B) | TYPE: | amino acid |
| | | |
| (C) | TOPOLOGY: | linear |

(ii) MOLECULE TYPE: peptide
(iii) FRAGMENT TYPE: internal fragment
(iv) ORIGINAL SOURCE:

| | |
|---|---|
| ORGANISM: | *Gluconobacter suboxydans* |
| | |
| STRAIN: | IFO 3255 |

(v) FEATURE:
SEQUENCING METHOD: E

Lys Ser Gln Ser Tyr Phe Ala Gly Pro Ser Gln Tyr Ala Pro Gln
1               5                   10                  15
Ala Pro Gly Val Asn Ala Xaa Asn Leu
                20                  24

INFORMATION FOR SEQ ID NO:6:
(i) SEQUENCE CHARACTERISTICS:

| | | |
|---|---|---|
| (A) | LENGTH: | 16 residues |
| | | |
| (B) | TYPE: | amino acid |
| | | |
| (C) | TOPOLOGY: | linear |

(ii) MOLECULE TYPE: peptide
(iii) FRAGMENT TYPE: internal fragment
(iv) ORIGINAL SOURCE:

| | |
|---|---|
| ORGANISM: | *Gluconobacter suboxydans* |
| | |
| STRAIN: | IFO 3255 |

(v) FEATURE:
SEQUENCING METHOD: E

Lys Val Leu Trp Gln His Pro Leu Gly Thr Ala Arg Xaa Asn Gly
1               5                   10                  15
Pro
16

INFORMATION FOR SEQ ID NO:7:
(i) SEQUENCE CHARACTERISTICS:

| | | |
|---|---|---|
| (A) | LENGTH: | 30 residues |
| | | |
| (B) | TYPE: | amino acid |
| | | |
| (C) | TOPOLOGY: | linear |

(ii) MOLECULE TYPE: peptide
(iii) FRAGMENT TYPE: N-terminal fragment
(iv) ORIGINAL SOURCE:

| | |
|---|---|
| ORGANISM: | *Gluconobacter suboxydans* |
| | |
| STRAIN: | IFO 3255 |

(v) FEATURE:
SEQUENCING METHOD: E

Gln Phe Ala Pro Ala Gly Ala Gly Gly Glu Pro Ser Ser Ser Val
1               5                   10                  15
Pro Gly Pro Gly Asn Ala Ser Glu Pro Thr Glu Asn Ser Pro Lys
                20                  25                  30

INFORMATION FOR SEQ ID NO:8:
(i) SEQUENCE CHARACTERISTICS:

| | | |
|---|---|---|
| (A) | LENGTH: | 24 residues |
| | | |
| (B) | TYPE: | amino acid |
| | | |
| (C) | TOPOLOGY: | linear |

(ii) MOLECULE TYPE: peptide
(iii) ORIGINAL SOURCE:

| | |
|---|---|
| ORGANISM: | *Gluconobacter suboxydans* |
| | |
| STRAIN: | IFO 3255 |

(iv) FEATURE:

| | |
|---|---|
| FEATURE KEY: | sig peptide |
| | |
| POSITION: | 1..24 |
| | |
| SEQUENCING METHOD: | E |

Met Arg Arg Pro Tyr Leu Leu Ala Thr Ala Ala Gly Leu Ala Leu
1               5                   10                  15
Ala Cys Ser Pro Leu Ile Ala His Ala
                20              24

INFORMATION FOR SEQ ID NO:9:
(i) SEQUENCE CHARACTERISTICS:

| | | |
|---|---|---|
| (A) | LENGTH: | 6 residues |
| | | |
| (B) | TYPE: | amino acid |
| | | |
| (C) | TOPOLOGY: | linear |

(ii) MOLECULE TYPE: peptide
(iii) FRAGMENT TYPE: C-terminal fragment
(iv) ORIGINAL SOURCE:

| | |
|---|---|
| ORGANISM: | *Gluconobacter suboxydans* |
| | |
| STRAIN: | IFO 3255 |

(v) FEATURE:
SEQUENCING METHOD: E

Pro Asp Ala Ile Lys Gln
1               5   6

## Claims

1. DNA encoding a protein having D-sorbitol dehydrogenase activity when cloned into an acetic acid bacterium, hybridisable under stringent conditions, with a DNA according to SEQ ID NO: 1.

2. DNA encoding a protein functional in activating D-sorbitol dehydrogenase in vivo, hybridisable under stringent conditions, with a DNA according to SEQ ID NO: 1.

3. An expression vector comprising a DNA according to claims 1 and/or 2.

4. Host cell transformed with a DNA according to claims 1 and/or 2 or a vector according to claim 3.

5. Host cell according to claim 4 selected from the group consisting of acetic acid bacteria including the genus Gluconobacter and Acetobacter.

6. A process for producing recombinant D-sorbitol dehydrogenase comprising the steps of cultivating a host cell comprising a DNA according to claims 1 and 2 in an appropriate medium, and recovering a protein with D-sorbitol dehydrogenase activity from the culture.

7. A process which comprises converting D-sorbitol into L-sorbose with the aid of a protein encoded by a DNA according to claim 1 and 2 or with a host cell comprising a DNA according to claim 1 and 2.

8. A process according to claim 7 wherein said L-sorbose is further converted into 2-KGA and/or vitamin C.

## Patentansprüche

1. DNA, codierend für ein Protein mit D-Sorbitoldehydrogenase-Aktivität bei Klonierung in ein Essigsäurebakterium, hybridisierbar unter stringenten Bedingungen mit einer DNA gemäß SEQ ID NO: 1.

2. DNA, codierend für ein bei der Aktivierung von D-Sorbitoldehydrogenase in vivo funktionelles Protein, hybridisierbar unter stringenten Bedingungen mit einer DNA gemäß SEQ ID NO: 1.

3. Expressionsvektor, umfassend eine DNA nach Anspruch 1 und/oder 2.

4. wirtszelle, transformiert mit einer DNA nach Anspruch 1 und/oder 2 oder einem Vektor nach Anspruch 3.

5. Wirtszelle nach Anspruch 4, ausgewählt aus der Gruppe Essigsäurebakterien, einschließlich der Gattungen Gluconobacter und Acetobacter.

6. Verfahren zur Herstellung rekombinanter D-Sorbitoldehydrogenase, umfassend die Schritte der Kultivierung einer eine DNA nach Anspruch 1 und 2 umfassenden Wirtszelle in einem geeigneten Medium und der Gewinnung eines Proteins mit D-Sorbitoldehydrogenase-Aktivität aus der Kultur.

7. Verfahren, umfassend die Umwandlung von D-Sozbitol mittels eines von einer DNA nach Anspruch 1 und 2 codierten Proteins oder mit einer eine DNA nach Anspruch 1 und 2 umfassenden Wirtszelle in L-Sorbose.

8. Verfahren nach Anspruch 7, wobei die L-Sorbose weiter in 2-KGA und/oder Vitamin C umgewandelt wird.

## Revendications

1. ADN codant pour une protéine ayant une activité D-sorbitol déshydrogénase lorsqu'il est cloné dans une bactérie acétique, pouvant s'hybrider dans des conditions stringentes avec un ADN selon SEQ ID NO : 1.

2. ADN codant pour une protéine fonctionnelle dans l'activation de la D-sorbitol déshydrogénase in vivo, pouvant s'hybrider dans des conditions stringentes avec un ADN selon SEQ ID NO : 1.

3. Vecteur d'expression comprenant un ADN selon les revendications 1 et/ou 2.

4. Cellule hôte transformée par un ADN selon les revendications 1 et/ou 2 ou un vecteur selon la revendication 3.

5. Cellule hôte selon la revendication 4, choisie dans le groupe constitué de bactéries acétiques y compris les genres Gluconobacter et Acetobacter.

6. Procédé de production de D-sorbitol déshydrogénase recombinante comprenant les étapes consistant à mettre en culture une cellule hôte comprenant un ADN selon les revendications 1 et 2 dans un milieu approprié, et à récupérer une protéine ayant une activité D-sorbitol déshydrogénase à partir de la culture.

7. Procédé qui comprend l'étape consistant à transformer du D-sorbitol en L-sorbose à l'aide d'une protéine codée par un ADN selon les revendications 1 et 2 ou d'une cellule hôte comprenant un ADN selon les revendications 1 et 2.

8. Procédé selon la revendication 7, selon lequel ledit L-sorbose est transformé plus avant en 2-KGA et/ou en vitamine C.
